# EUROPÄISCHE PATENTANMELDUNG

(11) **EP 0 705 830 A1**
(43) Veröffentlichungstag der Anmeldung: **10.04.1996**
(21) Anmeldenummer: 95112764.6
(22) Anmeldetag: 14.08.1995
(51) Int. Cl.: C07D 471/00, A61K 31/44, C07D 491/048

(54) **2,3-Cyclisch kondensierte 1,4-Dihydropyridine, Verfahren zu ihrer Herstellung und ihre Verwendung als selektive Kaliumkanalmodulatoren**

(30) Priorität: 25.08.1994 DE 4430092
(71) Anmelder: BAYER AG, D-51368 Leverkusen (DE)
(72) Erfinder: Urbahns, Klaus, Dr., D-42115 Wuppertal (DE); Goldmann, Siegfried, Dr., D-42327 Wuppertal (DE); Heine, Hans-Georg, Dr., D-47800 Krefeld (DE); Junge, Bodo, Dr., D-42399 Wuppertal (DE); Schohe-Loop, Rudolf, Dr., D-42327 Wuppertal (DE); Sommermeyer, Henning, Dr., D-51061 Köln (DE); Glaser, Thomas, Dr., D-51491 Overath (DE); Wittka, Reilinde, Dr., D-51069 Köln (DE); de Vry, Jean-Marie-Viktor, Dr., D-51503 Rösrath (DE)

(57) **Zusammenfassung**

Die vorliegende Erfindung betrifft neue 2,3-cyclisch kondensierte 1,4-Dihydropyridine der allgemeinen Formel (I), 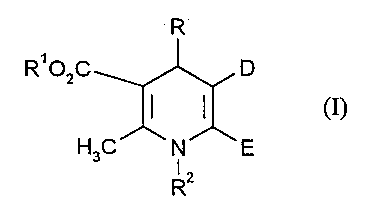
in welcher R, R¹, R², D und E die in der Beschreibung angegebene Bedeutung haben, Verfahren zu ihrer Herstellung und ihre Verwendung als Arzneimittel, vorzugsweise zur Behandlung des zentralen Nervensystems.

## Beschreibung

Die vorliegende Erfindung betrifft neue 2,3-cyclisch kondensierte 1,4-Dihydropyridine, Verfahren zu ihrer Herstellung und ihre Verwendung als Arzneimittel, vorzugsweise zur Behandlung des zentralen Nervensysstems.

Aus der Publikation DE 20 031 48 sind bereits 1,4,5,6,7,8-hexahydro-5-oxochinoline und 1,2,3,4,5,6,7,8,9,10-decahydro-1,8-dioxoacridine mit einer spasmolytischen und antihypertensiven Wirkung bekannt.

Außerdem sind 1-substituierte 1,4-Dihydropyridinderivate mit einer cerebralen durchblutungssteigernden Wirkung bekannt [vgl. DE 23 028 66].

Gegenstand der Erfindung sind neue 2,3-cyclisch kondensierte 1,4-Dihydropyridine der allgemeinen Formel (I), 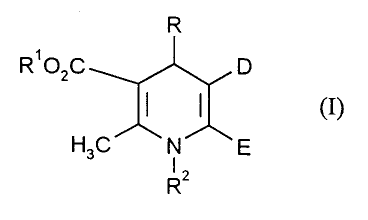
in welcher
- R: für Aryl mit 6 bis 10 Kohlenstoffatomen oder Pyridyl steht, die gegebenenfalls bis zu 5-fach gleich oder verschieden durch Cyano, Halogen, Trifluormethyl oder durch geradkettiges oder verzweigtes Alkylthio mit bis zu 6 Kohlenstoffatomen substituiert sind,
- R¹: für Wasserstoff oder für geradkettiges oder verzweigtes Alkyl mit bis zu 8 Kohlenstoffatomen steht,
- R²: für geradkettiges oder verzweigtes Alkyl mit bis zu 6 Kohlenstoffatomen oder für Cycloalkyl mit 3 bis 6 Kohlenstoffatomen steht,
- D und E: gemeinsam für einen bivalenten Rest der Formel -CO-(CH₂)₃-, -CO-CH₂-C(CH₃)₂-CH₂-, -CO-O-CH₂- oder -CH₂-O-CO- stehen,
und deren Salze.

Die Erfindungen zeigen überraschenderweise eine spezifische und selektive, modulierende Wirkung auf Kaliumkanäle und sind somit geeignet zur Verwendung als Arzneimittel, insbesondere als Mittel zur Bekämpfung von Erkrankungen des zentralen Nervensystems und der Sichelzellenanämie.

Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Mäleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen

Bevorzugt sind Verbindungen der allgemeinen Formel (I),
in welcher
- R: für Phenyl, Naphthyl oder Pyridyl steht, die gegebenenfalls bis zu 3-fach gleich oder verschieden durch Cyano, Fluor, Chlor, Brom, Iod, Trifluormethyl oder durch geradkettiges oder verzweigtes Alkylthio mit bis zu 4 Kohlenstoffatomen substituiert sind,
- R¹: für Wasserstoff oder für geradkettiges oder verzweigtes Alkyl mit bis zu 6 Kohlenstoffatomen steht,
- R²: für geradkettiges oder verzweigtes Alkyl mit bis zu 4 Kohlenstoffatomen, Cyclopropyl, Cyclopentyl oder Cyclohexyl steht,
- D und E: gemeinsam für einen Rest der Formel -CO-(CH₂)₃-, -CO-CH₂-C(CH₃)₂-CH₂-, -CO-O-CH₂- oder -CH₂-O-CO- stehen,
und deren Salze.

Besonders bevorzugt sind Verbindungen der allgemeinen Formel (I),
in welcher
- R: für Phenyl oder Pyridyl steht, die gegebenenfalls bis zu 2-fach gleich oder verschieden durch Cyano, Fluor, Chlor, Trifluormethyl oder durch Methylthio substituiert sind,
- R¹: für geradkettiges oder verzweigtes Alkyl mit bis zu 4 Kohlenstoffatomen steht,
- R²: für Methyl, Ethyl oder Cyclopropyl steht,
- D und E: gemeinsam für einen Rest der Formel -CO-(CH₂)₃-, -CO-CH₂-C(CH₃)₂-CH₂-, -CO-O-CH₂- oder -CH₂-O-CO- stehen,
und deren Salze.

Die erfindungsgemäßen Verbindungen der allgemeinen Formel (I) zeigen ein nicht vorhersehbares, wertvolles pharmakologisches Wirkspektrum.

Sie sind Kanalmodulatoren mit einer überraschenden Selektivität für Calciumabhängige Kalium-Kanäle großer Leitfähigkeit (BK(Ca)-Kanäle), insbesondere Kalium-Kanal Modulatoren des zentralen Nervensystems. Gleichzeitig zeichnen sie sich aus durch das Fehlen signifikanter Calcium-antagonistischen und Calciumagonistischer Wirkungen.

Aufgrund ihrer pharmakologischen Eigenschalten können sie für die Herstellung von Arzneimitteln zur Behandlung von zentral degenerativen Erkrankungen eingesetzt werden, wie z.B. bei Auftreten von Demenzen (Multiinfarktdemenz (MID), primär degenerativer Demenz (PDD), prä- und seniler Alzheimerscher Krankheit, HIV-Demenz und andere Demenzformen), Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe, Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten. Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Arrhythmie, Angina und Diabetes.

Die erfindungsgemäßen Verbindungen der allgemeinen Formel (I) können hergestellt werden, indem man
[A] im Fall D/E ≠ -CH₂-O-CO- Aldehyde der allgemeinen Formel (II)

   R-CHO (II)

   in welcher
   - R: den oben aufgeführten Bedeutungsumfang umfaßt,
   zunächst durch Umsetzung mit Verbindungen der allgemeinen Formeln (III) und (IV) 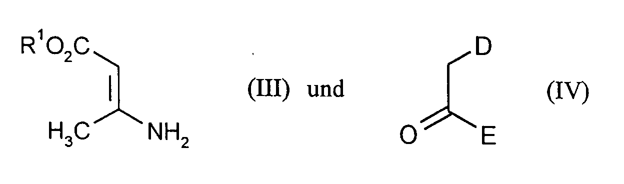 in welcher
   R¹, D und E die oben angegebene Bedeutung haben R¹ aber nicht für Wasserstoff steht,
   in inerten Lösemitteln und gegebenenfalls in Anwesenheit einer Base und/oder Hilfsmittels in die Verbindungen der allgemeinen Formel (V) 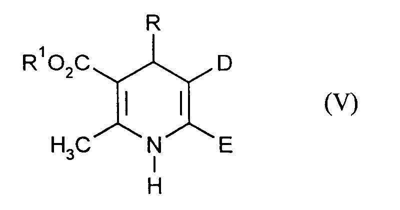 in welcher
   R, R¹, D und E die oben angegebene Bedeutung haben,
   überführt,
   und anschließend mit Alkylierungsmitteln der Formel (VI)

   R²-X (VI)

   in welcher
   - R²: die oben angegebene Bedeutung hat,
   und
   - X: für Halogen, vorzugsweise für Jod steht,
   nach üblichen Methoden umsetzt,
   oder
[B] im Fall, daß D/E für den Rest der Formel -CH₂-O-CO- stehen
   2-Amino-2-buten-4-olid der Formel (VII) 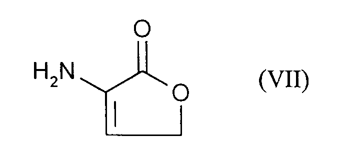 mit Benzylidenverbindungen der allgemeinen Formel (VIII) 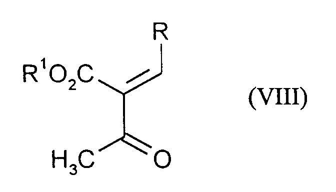 in welcher
   R und R¹ die oben angegebene Bedeutung haben,
   in inerten Lösemitteln, in Anwesenheit von Essigsäure zu Verbindungen der allgemeinen Formel (IX) 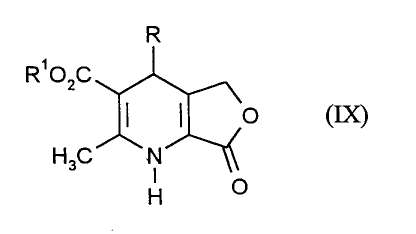 in welcher
   R und R¹ die oben angegebene Bedeutung haben,
   umsetzt und anschließend nach üblichen Mitteln eine Alkylierung durchführt,
   und im Fall R¹ = H die Ester nach üblichen Methoden hydrolysiert.

Das erfindungsgemäße Verfahren kann durch folgendes Formelschema beispielhaft erläutert werden: 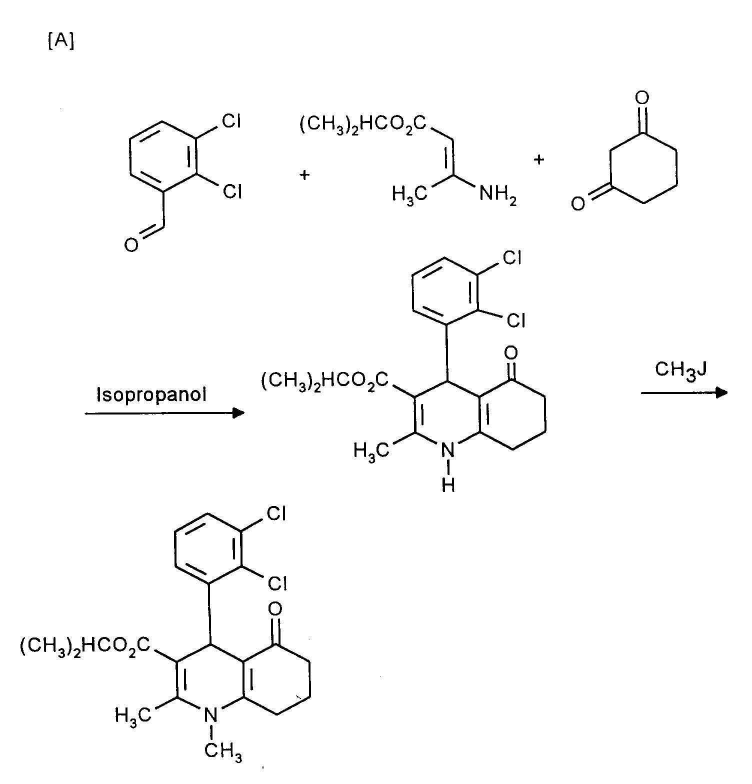 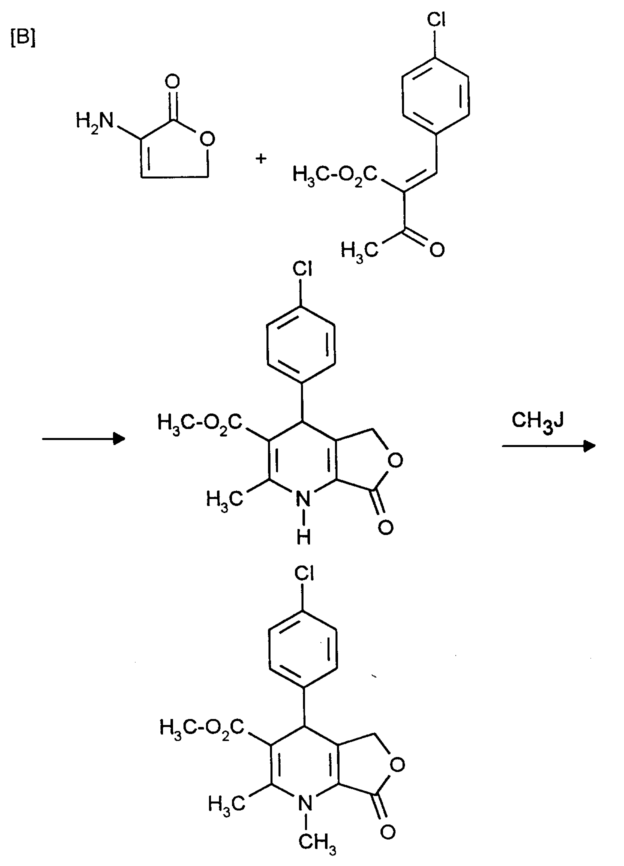
Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, Aceton oder Amide wie Hexamethylphosphorsäuretriamid oder Dimethylformamid, oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt sind Isopropanol, Ethanol, Tetrahydrofuran, Methanol, Dioxan, Aceton und Dimethylformamid.

Als Basen eignen sich im allgemeinen Alkalihydride, -carbonate oder -alkoholate, wie beispielsweise Natriumhydrid, Kaliumcarbonat oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind in Abhängigkeit der jeweiligen Reaktionsschritte Piperidin, Dimethylaminopyridin, Pyridin, Natriumhydrid, Kalium-tert.butylat und Kaliumcarbonat.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethysilfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen eignen sich im allgemeinen Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Natriumhydrid.

Die Reaktionstemperaturen können in einem größeren Bereich variiert werden. Im allgemeinen arbeitet man zwischen -10°C und +150°C, vorzugsweise zwischen +20°C und +100°C, insbesondere bei Raumtemperatur.

Die Alkylierung wird in den oben aufgeführten Lösemitteln bei Temperaturen von -5°C bis +150° C, vorzugsweise bei 0°C bis +100°C durchgeführt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Base wird im allgemeinen in einer Menge von 1 mol bis 3 mol, bevorzugt von 1 mol bis 2 mol jeweils bezogen auf 1 mol der zu alkylierenden Verbindungen eingesetzt.

Enantiomerenreine Formen erhält man z.B., indem man Diastereomerengemische der Verbindungen der allgemeinen Formel (I), in welcher R¹/R^{1'} für einen optisch aktiven Estertest steht, nach üblicher Methode trennt, anschließend entweder direkt umestert oder zuerst die chiralen Carbonsäuren herstellt und dann durch Veresterung die enantiomerenreinen Dihydropyridine herstellt.

Die Trennung der Diastereomeren erfolgt im allgemeinen entweder durch fraktionierte Kristallisation,durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formeln (II), (III), (IV), (V), (VI), (VII) und (VIII) sind bekannt oder können nach üblichen Methoden hergestellt werden.

Die erfindungsgemäßen neuen Verbindungen der allgemeinen Formel (I) zeigen ein wertvolles, nicht vorhersehbares Wirkspektrum, insbesondere auf Grund ihrer Selektivität auf Calcium-abhängige Kalium-Kanäle mit großer Leitfähigkeit.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Aus den Daten wird die durch Ionomycin hervorgerufene Erhöhung des Effluxes über den Basalefflux berechnet und als 100 % gesetzt. Die Stimulationen in Gegenwart von Prüfsubstanzen werden dann auf diesen Wert bezogen.

### Testdaten

### Beispiel 1

4-(2,3-Dichlorphenyl)-1,2-dimethyl-5-oxo-1,4,5,7,8-hexahydrochinolin-3-carbonsäureisopropylester
38 % Residualefflux bei 1 µM ,eingesetzter Prüfsubstanz.

### Beispiel 8

1,4,5,7-Tetrahydro-4-(4-chlorphenyl)-1,2-dimethyl-7-oxo-furo-[3,4b]-pyridin-3-carbonsäure-methylester
53 % Residualefflux bei 1 µM eingesetzter Prüfsubstanz.

### Beispiel 11

1,4,5,7-Tetrahydro-4-(2,3-difluorphenyl)-1,2-dimethyl-5-oxo-furo-[3,4b]-pyridin-3-carbonsäure-methylester
73 % Residualefflux bei 1 µM eingesetzter Prüfsubstanz.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Laufmittelgemische:

- a :: Methylenchlorid / AcOEt 10+1
- b :: Methylenchlorid / MeOH 10+1
- c :: PE / AcOEt 7+3

### Ausgangsverbindungen

### Beispiel I

1,4,5,7-Tetrahydro-4-(4-chlorphenyl)-2-methyl-5-oxo-furo-(3,4b)-pyridin-3-carbonsäuremethylester 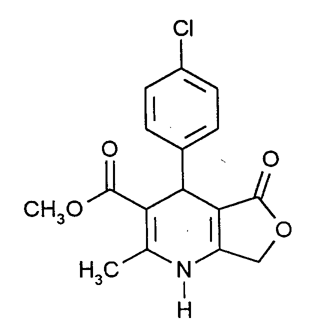
3 g (21,3 mmol) 4-Chlorbenzaldehyd, 4,0 g (21,3 mmol) 4-Acetoxyacetessigsäuremethylester (Herstellung nach DE 84 34436 78) und 2,5 g (21,3 mmol) 3-Aminocrotonsäuremethylester werden in 40 ml Isopropanol gelöst und 12 h zum Rückfluß erhitzt. Dann versetzt man mit 10 ml verdünnter wäßriger HCl und erhitzt weitere 30 min zum Rückfluß. Der Ansatz wird zwischen Toluol und Wasser verteilt. Trocknen (MgSO₄) und Einengen der organischen Phase liefert einen weißen Feststoff der durch Filtration über 50 g Kieselgel (AcOEt:PE 1+1) und anschließender Umkristallisation aus AcOEt/PE gereinigt wird. Man erhält 2,6 g (8,13 mmol, 38% d.Th.) der Titelverbindung.

### Beispiel II

4-(2,3-Dichlorphenyl)-2-methyl-5-oxo-1,4,5,7,8-hexahydrochinolin-3-carbonsäureisopropyleser 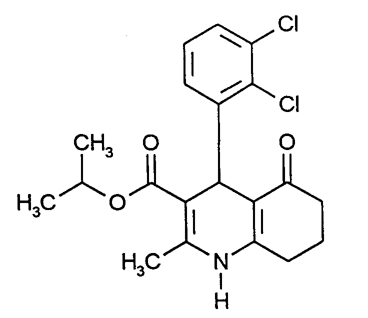
3,50 g (20 mmol) 2,3-Dichlorbenzaldehyd, 2,24 g (20 mmol) Dihydroresorcin, 2,86 g (20 mmol) 3-Aminocrotonsäureisopropylester werden in 100 ml Isopropanol gelöst und 5 h bei Rückfluß gerührt. Das Produkt fällt aus. Man versetzt mit 50 ml Wasser, und kühlt auf RT. Man saugt ab und wäscht nacheinander mit Isopropanol, Ethanol und Ether.
Ausbeute: 5,8 g (74% d.Th.)

### Beispiel III

2-Azido-γ-butyrolacton 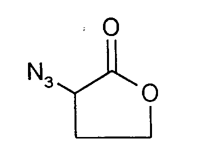
1,56 g (10 mmol) 2-Bromo-γ-butyrolacton werden in 2 ml Dimethylformamid gelöst und bei 0°C mit 612 mg (12,5 mmol) Lithiumazid versetzt. Man rührt 2 h bei Raumtemperatur, versetzt mit Wasser und extrahiert dreimal mit Methylenchlorid. Die vereinigten organischen Phasen werden dreimal mit Wasser gewaschen, über Natriumsulfat getrocknet und eingeengt. Man erhält 1,10 g (86,6% d.Th) der Titelverbindung.
MS : 127

### Beispiel IV

2-Amino-buten-4-olid 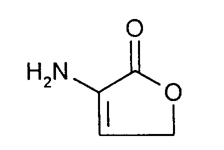
Zu einer Lösung aus 50 g Natrium in 5ml Ethanol tropft man bei 20°C 1,02 g (0,8 mmol) der Verbindung aus Beispiel III in 2 ml Ethanol hinzu. Man rührt 30 min bei Raumtemperatur und engt unter reduziertem Druck ein. Der ausgefallene Feststoff wird in wenig kaltem Ethanol ausgerührt und der Rückstand in heißem Essigsäureethylester gelöst. Die Lösung wird filtriert und eingeengt. Man erhält 350 mg (44% d.Th.) farblosen Feststoff.
MS: 99

### Beispiel V

1,4,5,7-Tetrahydro-4-(2,3-dichlorphenyl)-2-methyl-7-oxo-furo[3,4-b]pyridin-3-carbonsäureisopropylester 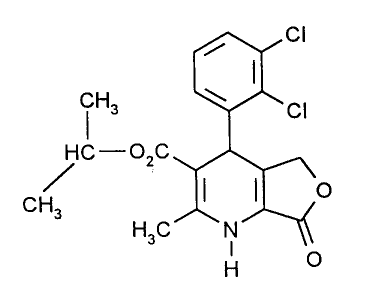
9,0 g (30 mmol) 2-Acetyl-3-(2,3-dichlorphenyl)acrylsäureisopropylester und 3,0 g (30 mmol) der Verbindung aus Beispiel IV werden in 60 ml Isopropanol gelöst und mit 1,7 ml (30 mmol) AcOH versetzt. Man hält 20 h bei Rückfluß. Anschließend wird eingeengt und der Rückstand über 100 g Kieselgel 60 gereinigt (Essigsäureethylester / Petrolether 10:1, dann 5:1). Das resultierende Material wird aus Ether umkristallisiert. Man erhält 4,08 g (36% d.Th.) der Titelverbindung.
MS: 381, Rf = 0,61 (Petrolether/AcOEt = 7 + 3)

### Herstellungsbeispiele

### Beispiel 1

4-(2,3-Dichlorphenyl)-1,2-dimethyl-5-oxo-1,4,5,7,8-hexahydrochinolin-3-carbonsäureisopropylester 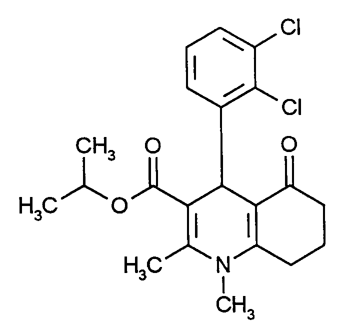
1,5 g (3,8 mmol) 4-(2,3-Dichlorphenyl)-2-methyl-5-oxo-1,4,5,7,8-hexahydrochinolin-3-carbonsäureisopropylester werden in 20 ml DMF gelöst und mit 138 mg (4,6 mmol) NaH bei 0°C versetzt. Man läßt 15 min bei Raumtemperatur rühren, gibt 1,08 g (7,6 mmol) Methyliodid bei 0°C zu und rührt erneut 30 min bei Raumtemperatur. Man versetzt mit Wasser und extrahiert 3 mal mit AcOEt. Die organische Phase wird 5mal mit Wasser gewaschen, getrocknet und eingeengt. Der Rückstand wird aus Isopropanol/Petrolether umkristallisiert.
Man erhält 1,20 g (77% d. Th.). MS: 407, Rf = 0,52 (a)
In Analogie zur Vorschrift des Beispiels 1 werden die in Tabelle 1 aufgeführten Verbindungen hergestellt:

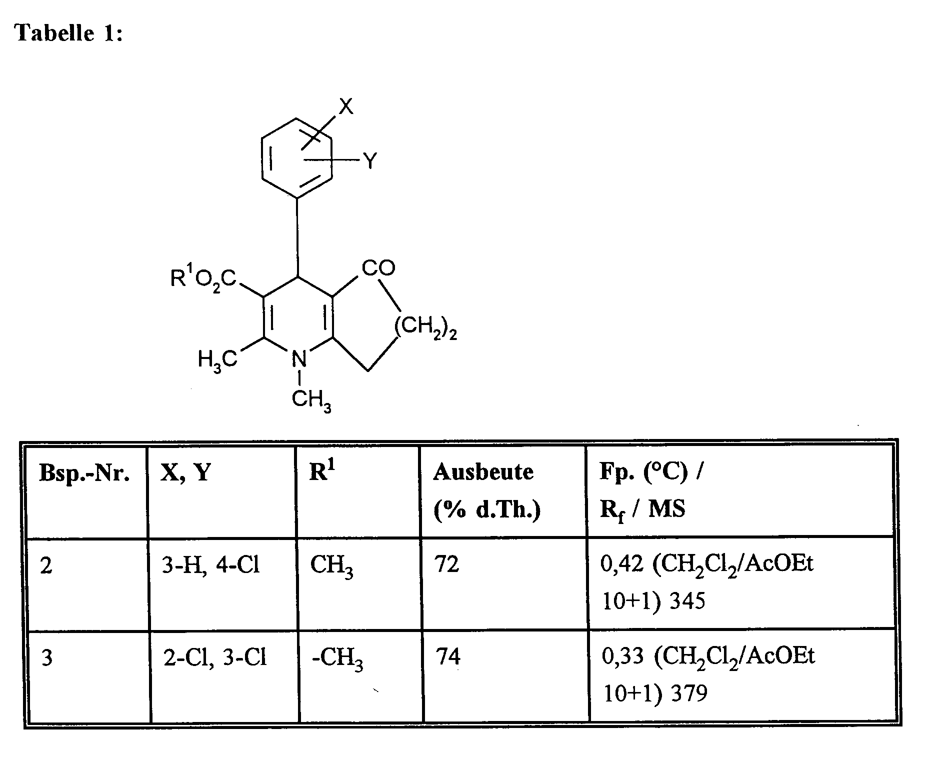

### Beispiel 4

4-(2,3-Dichlorphenyl)-1,2,7,7-tetramethyl-5-oxo-1,4,5,6,7,8-hexahydrochinolin-3-carbonsäure-isopropylester 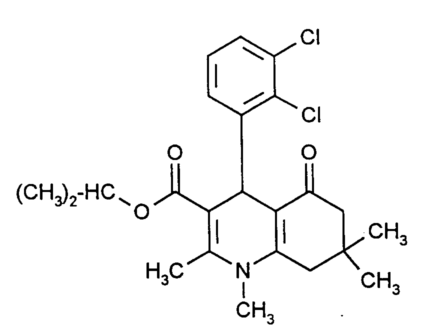
Analog der Vorschrift für Beispiel 1 werden aus 1,5 g (3,55 mol) 4-(2,3-Dichlorphenyl)-2-methyl-5-oxo-1,4,5,6,7,8-hexahydrochinolin-3-carbonsäure-isopropylester durch Methylierung mit 1,04 g (7,14 mol) Methyliodid in DMF 0,98 g (63% d.Th.) der Titelverbindung gewonnen.
Ausbeute: 60% d.Th.
Rf = 0,59 (CH₂Cl₂ / AcOEt = 10+1), MS: 435
In Analogie zur Vorschrift des Beispiels 4 werden die in Tabelle 2 aufgeführten Verbindungen hergestellt:

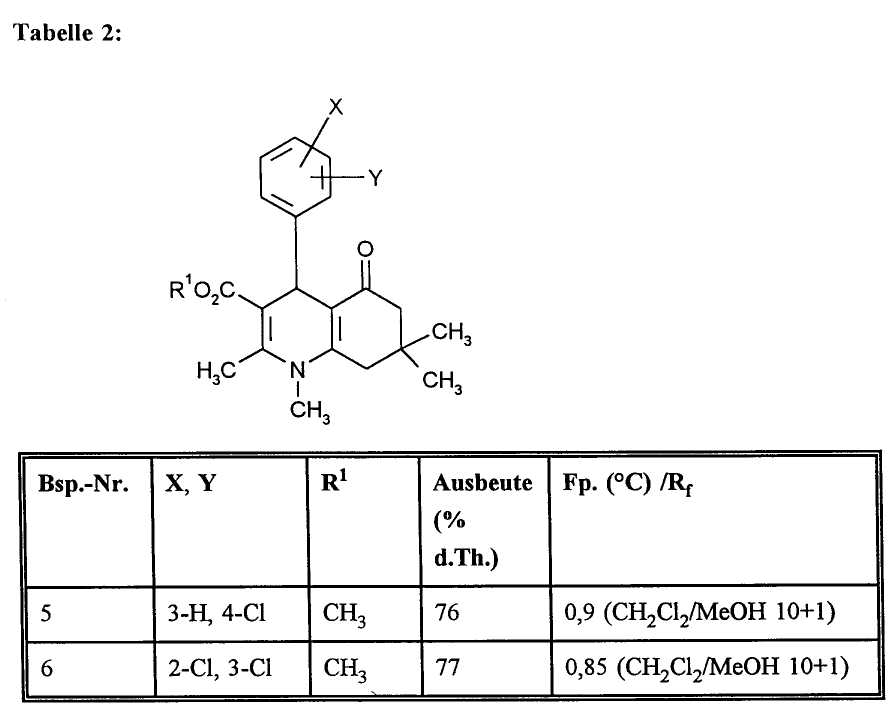

### Beispiel 7

1,4,5,7-Tetrahydro-4-(3,4-dichlorphenyl)-1,2-dimethyl-5-oxo-furo[3,4-b]pyridin-3-carbonsäure-isopropylester 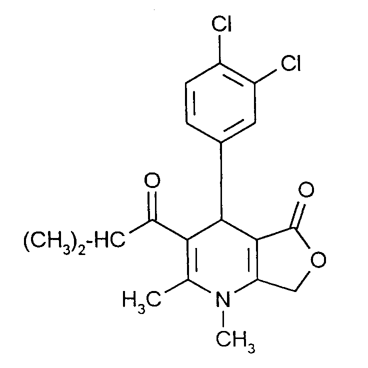
Analog der Vorschrift für Beispiel 1 werden aus 2,0 g (5,24 mol) 1,4,5,7-Tetrahydro-4-(4-chlorphenyl)-2-methyl-5-oxo-furo[3,4-b]pyridin-3-carbonsaure-methylester durch Methylierung mit 1,47 g (10,4 mol) Methyliodid in DMF 1,07 g der Titelverbindung gewonnen.
Ausbeute: 29% d.Th.
R_{f} = 0,40 (CH₂Cl₂ / AcOEt 10+1), MS: 395
In Analogie zur Vorschrift des Beispiels 7 werden die in Tabelle 3 aufgeführten Verbindungen hergestellt:

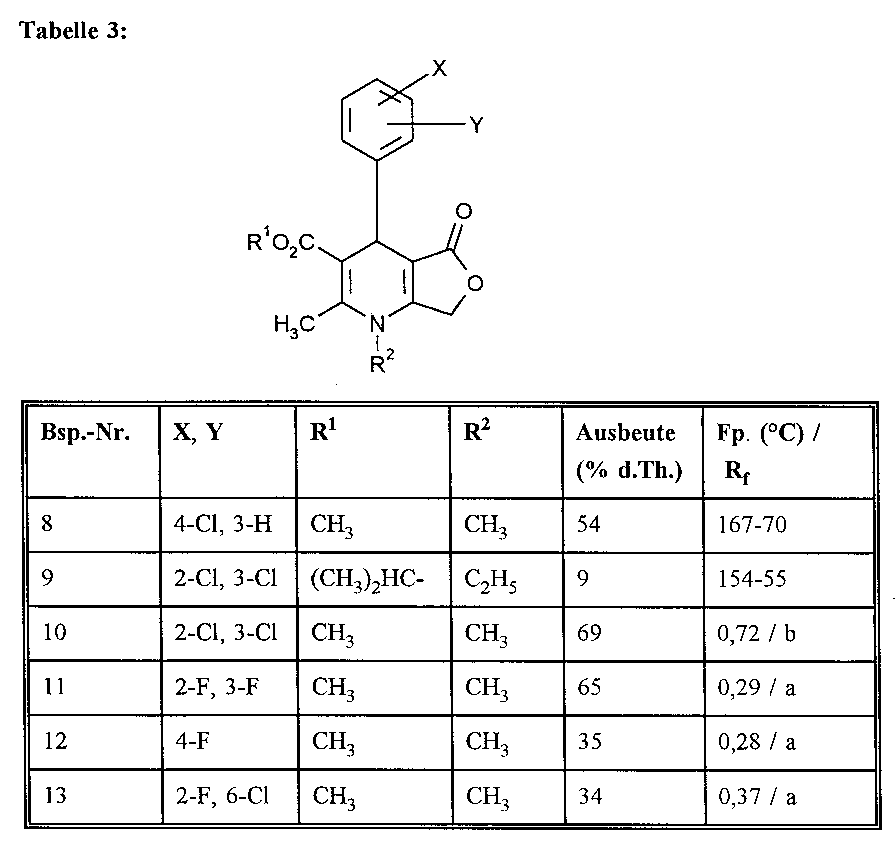

### Beispiel 14

1,4,5,7-Tetrahydro-4-(2,3-dichlorphenyl)-1,2-dimethyl-7-oxo-furo[3,4-b]-pyridin-3-carbonsäureisopropylester 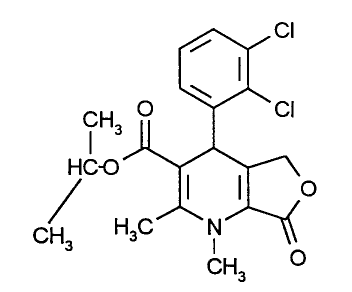
1,5 g (3,92 mmol) 1,4,5,7-Tetrahydro-4-(2,3-dichlorphenyl)-2-methyl-7-oxo-furo[3,4-b]pyridin-3-carbonsäureisopropylester werden mit 141 mg (4,70 mmol) NaH sowie 1,11 g (7,84 mmol) Methyliodid zu 0,97 g (63% d.Th.) umgesetzt.
MS: 395
R_{f} = 0,61 (PE / AcOEt = 7+3)
In Analogie zur Vorschrift des Beispiels 14 werden die in Tabelle 4 aufgeführten Verbindungen hergestellt:

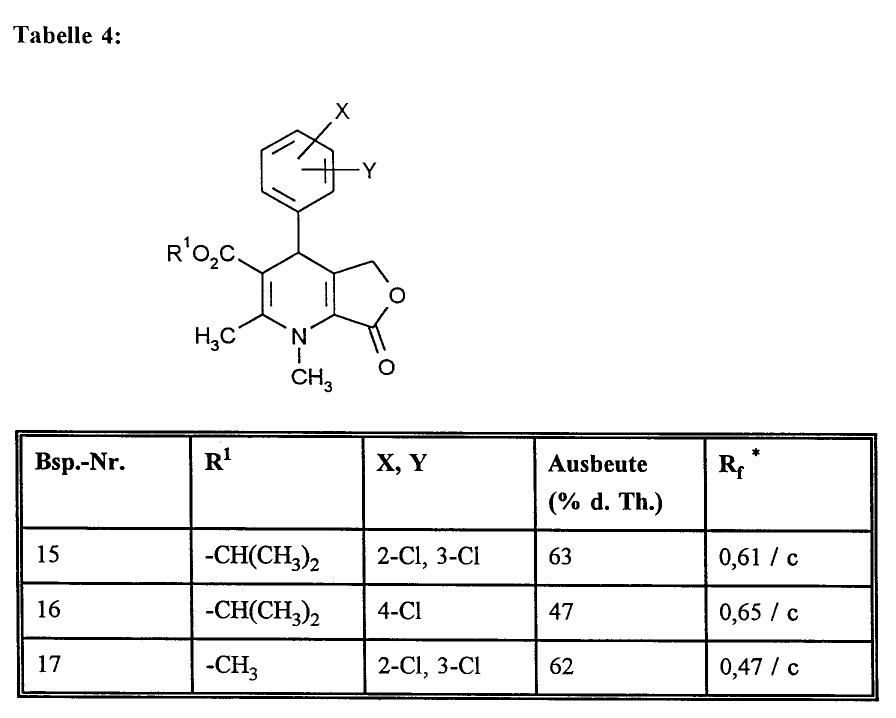

## Patentansprüche

1. 2,3-Cyclisch kondensierte 1,4-Dihydropyridine der allgemeinen Formel (I) 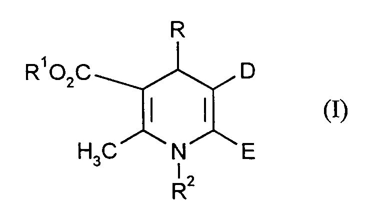 in welcher
R für Aryl mit 6 bis 10 Kohlenstoffatomen oder Pyridyl steht, die gegebenenfalls bis zu 5-fach gleich oder verschieden durch Cyano, Halogen, Trifluormethyl oder durch geradkettiges oder verzweigtes Alkylthio mit bis zu 6 Kohlenstoffatomen substituiert sind,
R¹ für Wasserstoff oder für geradkettiges oder verzweigtes Alkyl mit bis zu 8 Kohlenstoffatomen steht,
R² für geradkettiges oder verzweigtes Alkyl mit bis zu 6 Kohlenstoffatomen oder für Cycloalkyl mit 3 bis 6 Kohlenstoffatomen steht
D und E gemeinsam für einen bivalenten Rest der Formel -CO-(CH₂)₃-, -CO-CH₂-C(CH₃)₂-CH₂-, -CO-O-CH₂- oder -CH₂-O-CO- stehen,
und deren Salze.

2. Verbindungen der allgemeinen Formel (I) gemäß Anspruch 1 in welcher
R für Phenyl, Naphthyl oder Pyridyl steht, die gegebenenfalls bis zu 3-fach gleich oder verschieden durch Cyano, Fluor, Chlor, Brom, Iod, Trifluormethyl oder durch geradkettiges oder verzweigtes Alkylthio mit bis zu 4 Kohlenstoffatomen substituiert sind,
R¹ für Wasserstoff oder für geradkettiges oder verzweigtes Alkyl mit bis zu 6 Kohlenstoffatomen steht,
R² für geradkettiges oder verzweigtes Alkyl mit bis zu 4 Kohlenstoffatomen Cyclopropyl, Cyclopentyl oder Cyclohexyl steht,
D und E gemeinsam für einen Rest der Formel -CO-(CH₂)₃-, -CO-CH₂-C(CH₃)₂-CH₂-, -CO-O-CH₂- oder -CH₂-O-CO- stehen,
und deren Salze.

3. Verbindungen der allgemeinen Formel (I) gemäß Anspruch 1
in welcher
R für Phenyl oder Pyridyl steht, die gegebenenfalls bis zu 2-fach gleich oder verschieden durch Cyano, Fluor, Chlor, Trifluormethyl oder durch Methylthio substituiert sind,
R¹ für geradkettiges oder verzweigtes Alkyl mit bis zu 4 Kohlenstoffatomen steht,
R² für Methyl, Ethyl oder Cyclopropyl steht,
D und E gemeinsam für einen Rest der Formel -CO-(CH₂)₃-, -CO-CH₂-C(CH₃)₂-CH₂-, -CO-O-CH₂- oder -CH₂-O-CO- stehen,
und deren Salze.

4. Verfahren zur Herstellung von Verbindungen der allgemeinen Formel (I) gemäß Anspruch 1, dadurch gekennzeichnet, daß man
[A] im Fall D/E ≠ -CH₂-O-CO- Aldehyde der allgemeinen Formel (II)
R-CHO (II)
in welcher
R den oben aufgeführten Bedeutungsumfang umfaßt,
zunächst durch Umsetzung mit Verbindungen der allgemeinen Formeln (III) und (IV) 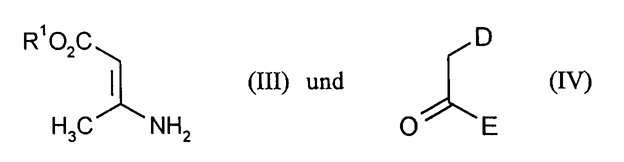 in welcher
R¹, D und E die oben angegebene Bedeutung haben, R¹ aber nicht für Wasserstoff steht,
in inerten Lösemitteln und gegebenenfalls in Anwesenheit einer Base und/oder Hilfsmittels in die Verbindungen der allgemeinen Formel (V) 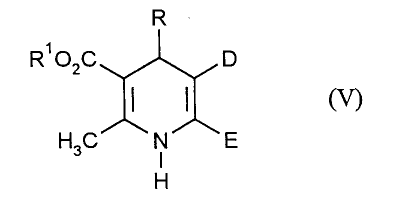 in welcher
R, R¹, D und E die oben angegebene Bedeutung haben,
überführt,
und anschließend mit Alkylierungsmitteln der Formel (VI)
R²-X (VI)
in welcher
R² die oben angegebene Bedeutung hat,
und
X für Halogen, vorzugsweise für Jod steht,
nach üblichen Methoden umsetzt,
oder
[B] im Fall, daß D/E für den Rest der Formel -CH₂-O-CO- stehen
2-Amino-buten-4-olid der Formel (VII) 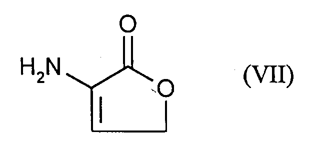 mit Benzylidenverbindungen der allgemeinen Formel (VIII) 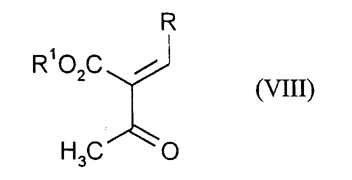 in welcher
R und R¹ die oben angegebene Bedeutung haben,
in inerten Lösemitteln, in Anwesenheit von Essigsäure zu Verbindungen der allgemeinen Formel (IX) 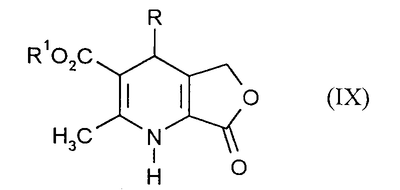 in welcher
R und R¹ die oben angegebene Bedeutung haben,
umsetzt und anschließend nach üblichen Mitteln eine Alkylierung durchführt,
und im Fall R¹ = H die Ester nach üblichen Methoden hydrolysiert.

5. Arzneimittel enthaltend mindestens eine Verbindung der allgemeinen Formel (I) gemäß Anspruch 1.

6. Verfahren zur Herstellung Von Arzneimitteln, dadurch gekennzeichnet, daß man mindestens eine Verbindung der allgemeinen Formel (I) gemäß Anspruch 1 gegebenenfalls mit üblichen Hilfs- und Trägerstoffen in eine geeignete Applikationsform überführt.

7. Verbindungen der allgemeinen Formel (I) gemäß Anspruch 1 zur Verwendung bei der Bekämpfung von Erkrankungen.

8. Verwendung von Verbindungen der allgemeinen Formel (I) gemäß Anspruch 1 bei der Herstellung von Arzneimitteln zur Behandlung des zentralen Nervensystems.
